# EUROPEAN PATENT SPECIFICATION

(11) **EP 2 916 853 B1**
(45) Date of publication and mention of the grant of the patent: **06.05.2020**
(21) Application number: 13788774.1
(22) Date of filing: 08.11.2013
(51) Int. Cl.: A61K 35/12, A61K 35/14, A61K 35/16, A61K 35/28, A61K 31/7105, A61K 31/713, A61K 9/00, A61M 15/00, A61P 11/00, A61P 11/06, A61P 35/00

(54) **COMPOSITIONS FOR PULMONARY DELIVERY**
ZUSAMMENSETZUNGEN ZUR PULMONALEN VERABREICHUNG
COMPOSITIONS POUR L'ADMINISTRATION PULMONAIRE

(30) Priority: 09.11.2012 DK 201270690
(43) Date of publication of application: 16.09.2015
(73) Proprietor: Velin-Pharma A/S, 3480 Fredensborg (DK)
(72) Inventor: VELIN, Flemming, DK-3480 Fredensborg (DK)
(74) Representative: Inspicos P/S
(86) International application number: PCT/EP2013/073388
(87) International publication number: WO 2014/072468

(56) References cited:
- WO-A1-2007/121538
- WO-A1-2009/100029
- WO-A1-2010/028652
- WO-A1-2010/118979
- WO-A1-2011/029903
- WO-A2-2009/143453
- US-A1- 2006 292 162
- US-A1- 2012 087 988

## Description

### FIELD OF THE INVENTION

The present invention relates to compositions for treatment or for alleviating the symptoms of a disease or disorder by the inhalation of a blood serum aerosol to the respiratory system. The invention further relates to the treatment of diseases or disorders by the inhalation of compositions to the respiratory system of a subject.

### BACKGROUND OF THE INVENTION

Inflammation, a key component of the immune system, functions in both defence (physiological) and in pathophysiological events to maintain the homeostasis of tissues, organs and individual cells. Acute inflammation is a short-term process characterized by the classic signs of inflammation, i.e. swelling, redness, pain, heat, and loss of function, due to infiltration of tissues by plasma of several activated components such as interleukins, antibodies, hormones etc. and leukocytes. It occurs as long as the injurious stimulus is present and ceases once the stimulus has been removed. Chronic inflammation is a pathological condition characterized by concurrent active inflammation, tissue destruction, and attempts at repair. Chronically inflamed tissue is characterized by the infiltration of mononuclear immune cells (monocytes, macrophages, lymphocytes, dendritic cells and other plasma cells), tissue destruction, and attempts at healing, which include angiogenesis and fibrosis.

Without inflammation, wounds and infections would not be able to heal and progressive destruction of the tissue would threaten the survival of the organism. Inappropriate inflammation, on the other hand, can lead to various diseases, including but not limited to indications such as hay fever, atherosclerosis, neurodegenerative diseases such as Alzheimer's, cancer and rheumatoid arthritis. For these reasons, inflammation is tightly regulated by the body.

Mononuclear immune cells are under infectious conditions attracted to the site of infection in an attempt to eliminate the foreign pathogen through phagocytosis. Leukocytes and dendritic cells are here activated by the pathogens to synthesize and release proinflammatory cytokines such as IL- 1α, IL- 1β, IL- 3, IL-5, IL-6, IL-8, TNF-α (tumor necrosis factor-α), GM-CSF (granulocyte-macrophage colony-stimulating factor), NF-κB (nuclear factor kappa-light-chain-enhancer of activated B cells), and MCP- I (monocyte chemotactic protein- 1). These released cytokines then further attract more immune cells to the infected site, amplifying the response of the immune system to defend the host against the foreign pathogen.

Dendritic cells are derived from hemopoietic bone marrow progenitor cells. These progenitor cells initially transform into immature dendritic cells. These cells are characterized by high endocytic activity and low T-cell activation potential. Immature dendritic cells constantly sample the surrounding environment for foreign pathogens. This is done through pattern recognition receptors such as the toll-like receptors (TLRs), which recognize specific chemical signatures found on subsets of pathogens. Once they have come into contact with a presentable antigen, they become activated into mature dendritic cells and begin to migrate to the lymph node. Immature dendritic cells phagocytize pathogens and degrade their proteins into small pieces and upon maturation present those fragments at their cell surface using MHC molecules. Simultaneously, they upregulate cell-surface receptors that act as co-receptors in T-cell activation. Once in the lymph nodes they act as antigen-presenting cells, in activating helper T-cells and killer T-cells as well as B-cells by presenting them with antigens derived from the pathogen, together with non-antigen specific co-stimulatory signals.

Glucocorticoids (also referred to as "corticosteroids" or "steroidal drugs") as well as Nonsteroidal Antiinflammatory Drugs (NSAIDs) represent today one of the most effective clinical treatment for a range of inflammatory conditions, including acute inflammation. However, these drugs can have side effects that may threaten the overall health of the patient.

The present inventors have found that body fluid compositions may be used in the treatment of inflammatory conditions, including acute inflammation and cancers. However, there remains to be developed safe, effective, faster and more convenient methods of treating autoimmune diseases, inflammatory disorders, and cancers or other indications associated with abnormal cell growth or cell division, such as indications associated with inflammation and cancers of the respiratory system. The present invention provides compositions for treating such diseases and disorders.

WO 2011/029903 relates to compositions comprising a therapeutically effective amount of miRNAs, their use for the treatment of medical conditions.

WO 2010/118979 relates to methods and compositions, such as activated blood serum preparations for use in the treatment of specific medical conditions.

WO 2009/143453 discloses compositions comprising purified plasma that includes concentrated levels of immunoglobulins and its use for treating lung inflammation.

### OBJECT OF THE INVENTION

It is an object of embodiments of the invention to provide compositions suitable for the treatment - by the inhalation of a body fluid composition to the respiratory system - of indications including but not limited limited to respiratory fibrosis, a respiratory hypersensitivity disorder, a cancer, such as lung cancer, non-small cell lung cancer (NSCLC), pulmonary hypertension, chronic obstructive lung disease (COLD), and asthma.

### SUMMARY OF THE INVENTION

It has been found by the present inventor(s) that body fluid compositions according to the present invention may be administered by inhalation with any suitable inhalation device in the treatment of various indications associated with the respiratory system.

The scope of the protection sought is defined by the claims. So, in a first aspect the present invention relates to a body fluid composition being a blood serum aerosol for use in the treatment or for alleviating the symptoms of a disease or disorder by the inhalation of the blood serum aerosol to the respiratory system.

Disclosed herein is also a method for the treatment or for alleviating the symptoms of a disease or disorder, the method comprising the inhalation of a body fluid composition to the respiratory system of a subject.

In an aspect the present invention relates to a body fluid composition for use according to the present invention, wherein the formulation is part of a kit comprising the composition and an inhalation device.

In a further aspect the present invention relates to a body fluid composition for use according to the present invention, wherein the formulation is comprised in an inhalation device.

Disclosed herein is further a composition comprising stem cells, such as adipose-derived stem cells for the treatment or for alleviating the symptoms of a disease or disorder by the inhalation of the body fluid composition to the respiratory system.

### DETAILED DISCLOSURE OF THE INVENTION

### Definitions

The term "body fluid composition" refers to any fluid that may be obtained from the body of a mammal or elements derived directly thereof. Included within this definition are cerebrospinal fluids, blood, such as blood from the circulatory system or from the umbilical cord, serum, lymph fluid, plasma, pleura exudates, peritoneal exudates, bone marrow exudates, extracellular fluids, fluids from the joints, amniotic fluids. Included within this definition are also cells, such hematopoietic stem cells or in vitro cell cultures, such as a monocyte cell cultures, as well as exosomes or other substructures that may be derived from a body fluid. Excluded from this definition are compositions consisting of one or two purified recombinant or synthetically produced therapeutically active steroids or proteins. Accordingly, the body fluid compositions according to the present invention are heterogeneous compositions comprising a mixture of different compounds.

The body fluid composition may be derived from blood or an element thereof, such as peripheral blood or any component derived from blood, such as a plasma composition or a serum composition. Such serum composition is prepared by a method comprising standard incubation of whole blood containing no anticoagulant followed by centrifugation to obtain a serum supernatant. In some embodiments the blood is incubation to be in contact with an increased surface for a specified period of time prior to incubation. In some embodiments, the serum composition is not purified with respect to any specific component of the serum. In some embodiments the body fluid composition according to the invention is a composition comprising erythrocytes and/or leukocytes, such as a composition, wherein the erythrocytes constitute more than about 40%, such as more that about 45%, 50%, 55%, 60%, 65%, 70%, 75%, 80%, 85%, 90%, 95%, 98% by volume of the composition. In some embodiments the body fluid composition is a composition

In some embodiments, the body fluid composition according to the invention is a composition comprising a therapeutically effective amount of one or more miRNA molecule or functional variant thereof, the miRNA being upregulated in a body fluid or element thereof upon activation of the body fluid or element thereof.

In some embodiments, the body fluid composition according to the invention is a composition comprising a therapeutically effective amount of one or more miRNA molecule or functional variant thereof, the miRNA being upregulated in a body fluid or element thereof upon activation of the body fluid or element thereof, the method comprising the steps of
a) Collecting the body fluid or element thereof from a mammal;
b) Incubating the collected body fluid or element thereof in contact with an increased surface area;
c) Collecting the body fluid produced after step b) and optionally purifying the miRNA.

In some embodiments, the body fluid or element thereof is incubated under step b) for more than 24 hours, such as more than 48 hours, such as for more than 60 hours, 72 hours, 84 hours, 96 hours, 120 hours, or 150 hours.

In some embodiments, the body fluid composition according to the invention is a composition comprising a therapeutically effective amount of one or more miRNA molecule or functional variant thereof, the miRNA being upregulated in a body fluid or element thereof upon activation of the body fluid or element thereof, the method comprising the steps of
a) Collecting the body fluid or element thereof from a mammal;
b) Incubating the collected body fluid or element thereof in contact with an increased surface area;
c) Identifying one or more miRNA upregulated in the body fluid or element thereof;
d) Providing the one or more miRNA molecule identified in step c) in isolated form and adding them to the composition.

The miRNA may be upregulated in a body fluid or element thereof upon activation in a vessel optionally comprising an inductor. In some embodiments the vessel has a wall structure formed continuously about an internal space and an entry point provided in a top end of the wall for injecting the body fluid or element thereof into the internal space.

The body fluid composition according to the invention may be prepared or conditioned in a device consisting of a 60 ml container suitable for centrifugation comprising two rubber ports for injection and a small hole for pressure equalization, the container being with or without 2 -25 gram of glass beads, 1-8 mm, such as 4mm. Other means than beads for increasing the surface area may be used.

Other potentially suitable devices to condition or to prepare the body fluid compositions according to the present invention are described in any one of EP0740964, EP1638691, WO2008097230, EP1093390, or EP1549552, the device being prefilled in the chamber for collection of supernatant with beads to stimulate the production of miRNA, or the chamber for collection of supernatant being provided with a surface structure which stimulates the production of miRNA.

The body fluid compositions according to the present invention may be an activated blood preparation, wherein the blood preparation is activated in a device as described herein or potentially in any one of EP0740964, EP1638691, WO2008097230, EP1093390, or EP1549552 (all of which are hereby incorporated by reference), the device being prefilled in the chamber for collection of supernatant with beads to stimulate the production of miRNA, or the chamber for collection of supernatant being provided with a surface structure which stimulates the production of miRNA.

Methods for preparing body fluid compositions according to the present invention are described in any one of WO 2010/118979 and WO 2010/118979, the content of which is hereby incorporated by reference.

In some embodiment the body fluid or element thereof may be activated in a device including but not limited to a device as disclosed in any one of international patent applications WO06007529, WO07090569, WO03080122, WO0046249, or WO9909051.

The term "inductor" or "enhancing agent" may be used interchangeably and is used herein to refer to any substance or compound that may be used to induce maturation or activation of the body fluid preparation used according to the invention, such in activation in antigen presenting cells (APCs). In some embodiments the inductor is a biological compound such as immunoglobulins or cancer cells that are able to induce or potentiate an immunological response in leukocytes or dendritic cells of the blood preparation.

The term "anticoagulant" as used herein refers to any substance that prevents coagulation. Included within this definition is Warfarin (Coumadin), Acenocoumarol, phenprocoumon, Phenindione, Heparin, Low molecular weight heparin, Synthetic inhibitors of factor Xa, such as Fondaparinux and Idraparinux, thrombin inhibitors, such as argatroban, lepirudin, bivalirudin, and dabigatran.

The term "Platelet-rich plasma" or "PRP", as used herein refers to a concentrated source of platelets, such as autologous platelets. PRP is known to contain and also releases (through degranulation) several growth factors (cytokines) that stimulate soft tissue healing.

In some embodiments, the body fluid composition may be an activated blood serum preparation that has been mixed with platelet-rich plasma (PRP). This may be used for several types of medical disorders or conditions, cardiac care, cartilage regeneration, disc regeneration, and dental health.

In some non-limiting embodiments of the invention, a body fluid composition is further activated to enhance the anti-inflammatory and/or immunosuppressive activity of the preparation.

Enhancing agents may be cytokines, cytokine antagonists, and NFkappaB antagonists, and include, but are not limited to, TGF-β, IL-10, CTLA4-Ig, sCD40-Ig, IL-4, IL-13, FasL, IL-1 receptor antagonist protein (IL-1Ra), vIL-10, sICAM-1, sICAM-3, and TRAIL. In some non-limiting embodiments, the enhancing agent is IL-1Ra, IL-10 or IL-4 or a combination thereof.

Optionally, where a specific antigen or a specific antigen source is known including but not limited to antigens derived from virus or bacteria, such specific antigen or specific antigen source (e.g., fixed or attenuated infectious agent) may be added to the culture as an enhancing agent.

In some embodiments of the invention, peripheral blood is removed from an individual, such as with a syringe, and thereafter transferred to a vessel, wherein the blood is activated by incubation in the presence of beads. Alternatively, the peripheral blood may be removed directly into a vessel comprising beads. Serum may then be collected and used according to the invention.

In still another alternative peripheral blood is removed from an individual, the buffy coat and/or serum is harvested from the blood preparation and transferred to a vessel comprising beads.

In still other alternatives peripheral blood, a preparation of bone marrow, or other preparation containing blood components is removed from an individual or obtained in other ways, transferred to a device as described herein or potentially in any one of EP0740964, EP1638691, WO2008097230, EP1093390, or EP1549552, the device being prefilled in the chamber for collection of supernatant with beads, or the wall of the chamber for collection of supernatant having a surface structure, including a clean surface, which may stimulate the production of miRNA.

Beads which may be used for this purpose include, but are not limited to, glass or plastic beads between 0.5 and 10 mm or between 0.5 and 5 mm in diameter, optionally treated with an agent, such as CrSO₄, which stimulates lymphocyte proliferation (Mignini et al., 2004, Preventive Med 39(4) 767-775; Rhee et al., 2002, Clin Exp Immunol 127(3):463-469). Medical grade glass beads, 4 mm in diameter may be modified by incubation in 50% CrSO₄. The treated or untreated beads are placed in a suitable container, such as a microtiter plate, centrifuge tube, culture tube, or syringe or other device described herein, and then sterilized (e.g. by autoclaving or gamma irradiation). Peripheral blood may in some embodiments be introduced into the bead-containing container, and then incubated, aseptically, at 37 °C., optionally with 5% CO₂, for example for 2-200 hours, such as for 2-150 hours or 2-48 hours. Serum may then be collected from the bead/blood suspension by centrifugation, for example at 4200 rpm for 10 minutes. Typically, 30 % of the total original peripheral blood volume may be recovered. The resulting serum may then be stored at -20 °C.

In a related, specific, non-limiting embodiment of the invention, enhancing agents may be added to the peripheral blood sample prior to, or as an alternative to, incubation with beads. For example, 5 µg enhancing agent per ml of peripheral blood may be added.

In some, non-limiting embodiment of the invention, a blood serum preparation is prepared by collecting serum from peripheral blood, optionally incubated with beads and/or an enhancing agent, by centrifugation to remove the formed blood elements (e.g., at 3000-5000 g for 10 minutes), followed by ultracentrifugation, for example, at 100,000 g, for 1 hours. The resulting pellet may be re-suspended in physiologic saline, and then preferably sterilized (e.g., by filtration through a 0.2 µm filter).

The blood may in some embodiments be treated with an anticoagulant, such as heparin or citrate.

In further embodiments the body fluid compositions according to the present invention is prepared by the incubation of the body fluid or elements thereof in the container to be carried out over a period of from 2 to 200 hours, such as 2-150 hours, such as 2-100, or 2 to 72 hours, such as 20 hours, preferably at physiological or room temperature, that is to say 20 °C to 41 °C, in particular at 35-37 °C.

The body fluid may be treated further after formation of the therapeutically or prophylactically active compounds in the body fluid, in order, for example, to remove particular constituents, for example blood plasma or blood platelets. This removal may in some embodiments of the invention be carried out by centrifugation, filtration or coagulation to remove coagulation factors and/or clotted material. In alternative embodiments of the invention the body fluid composition may be mixed with other body fluids or body fluid components. Accordingly, in some particular embodiments, an activated body fluid composition, such an activated blood serum preparation is mixed with Platelet-rich plasma prior to administration to the patient. This is particularly suitable for use in the treatment of indications associated with joints, and muscles, such as in the treatment of muscle pain, polymyalgia rheumatica.

In some aspects of the invention, the body fluid composition is a blood preparation activated in a container or by alternative methods. In some alternative embodiments the blood preparation is replaced with another body fluid and prepared as described elsewhere. Accordingly, in some alternative embodiments, a container is filled with a body fluid or elements thereof, such as in vitro cell cultures, bone marrow exudates and the like, and treated by methods similar or identical to methods used for treating blood preparations. In some embodiments the body fluid is taken with a syringe directly from the patient.

In some embodiments, the buffy coat is removed from a blood preparation and activated. This may be in the presence or absence of the plasma fraction of the blood preparation. In some embodiments the buffy coat with or without plasma is incubated in the presence of a growth medium, such as during the activation on a surface or prior to said activation.

In some embodiments the body fluid compositions according to the invention further comprise micelles, vesicles or liposomes, and preferably the micelles, vesicles, such as liposomes comprising miRNA.

Liposomes are typically completely closed structures comprising lipid bilayer membranes containing an encapsulated aqueous volume. Liposomes may contain many concentric lipid bilayers separated by an aqueous phase (multilamellar vesicles or MLVs), or alternatively, they may comprise a single membrane bilayer (unilamellar vesicles). The lipid bilayer is composed of two lipid monolayers having a hydrophobic "tail" region and a hydrophilic "head" region. In the membrane bilayer, the hydrophobic (nonpolar) "tails" of the lipid monolayers orient toward the centre of the bilayer, whereas the hydrophilic (polar) "heads" orient toward the aqueous phase. The lipid components that may be used in the liposomes described herein are generally described in the literature. Generally, these are phospholipids - such as phosphatidylcholine, phosphatidylethanolamine, phosphatidylglycerol, phosphatidylserine, phosphatidic acid, phosphatidylinositol and/or sphingolipids. Additional components, fore example, sterols - such as cholesterol - or other components - such as fatty acids (e.g., stearic acid, palmitic acid), dicetyl phosphate or cholesterol hemisuccinate, may be used. Moreover, the liposome membrane can also contain preservatives. The liposome membrane may also contain components, which modify their dispersion behaviour. They include, for example, PEGylated derivatives of phosphatidylethanolamine, lipids - such as GM 1 - or conjugates of sugars and hydrophobic components - such as palmitic or stearic acid esters of dextran.

The basic structure of liposomes may be made by a variety of techniques known in the art.

For example, liposomes have typically been prepared using the process of Bangham et al., (1965 J. MoI. Biol., 13: 238-252), whereby lipids suspended in organic solvent are evaporated under reduced pressure to a dry film in a reaction vessel. An appropriate amount of aqueous phase is then added to the vessel and the mixture agitated. The mixture is then allowed to stand, essentially undisturbed for a time sufficient for the multilamellar vesicles to form.

Liposomes may be reproducibly prepared using a number of currently available techniques. The types of liposomes which may be produced using a number of these techniques include small unilamellar vesicles (SUVs) [See Papahadjapoulous and Miller, Biochem. Biophys. Acta., 135, p. 624-638 (1967)], reverse-phase evaporation vesicles (REV) [See U.S. Pat. No. 4,235,871 issued Nov. 25, 1980], stable plurilamellar vesicles (SPLV) [See U.S. Pat. No. 4,522,803, issued June 11, 1985], and large unilamellar vesicles produced by an extrusion technique as described in copending U.S. patent application Ser. No. 622,690, filed June 20, 1984, Cullis et.al., entitled "Extrusion Technique for Producing Unilamellar Vesicles". In a preferred embodiment, the liposomes are prepared using the following method. The lipids are prepared by pipetting the appropriate amount of stock solutions of, for example, CDAN, DOPE and aminoxylipid (CPA), respectively, into a round bottomed flask pre-treated with nitric acid and dimethylsilyldichlorid, evaporating the solvent, and hydrating the dry lipid film with water under heavy vortexing, to generate multilamellar liposomes.

Unilamellar liposomes may be produced by sonication of the multilamellar liposomes for 30 minutes. Preferably, this is continued until a size of smaller than about 30nm is reached.

In some embodiments the body fluid compositions may be prepared to comprise therapeutically effective miRNA molecules or functional variants thereof. In some embodiments the body fluid compositions used according to the present invention comprises one or more miRNA selected from the group consisting of hsa-Let-7a, hsa-Let-7b, hsa-Let-7b*, hsa-Let-7c, hsa-Let-7d, hsa-Let-7d*, hsa-Let-7e, hsa-Let-7f , hsa-Let-7f*, hsa-Let-7g, hsa-Let-7g*, hsa-Let-7i, hsa-miR-103, hsa-miR-106A, hsa-miR-106B, hsa-miR-107, hsa-miR-125A, hsa-miR-125B, hsa-miR-126, hsa-miR-128, hsa-miR-130A, hsa-miR-130B, hsa-miR-140-3P, hsa-miR-140-5P, hsa-miR-142-3P, hsa-miR-142-5P, hsa-miR-143, hsa-miR-144, hsa-miR-146A, hsa-miR-148A, hsa-miR-148B, hsa-miR-150, hsa-miR-151-3P, hsa-miR-151-5P, hsa-miR-152, hsa-miR-15A, hsa-miR-15B, hsa-miR-16, hsa-miR-15B*, hsa-miR-17, hsa-miR-181A, hsa-miR-185, hsa-miR-186, hsa-miR-18A, hsa-miR-18A*, hsa-miR-18B, hsa-miR-192, hsa-miR-191, hsa-miR-194, hsa-miR-197, hsa-miR-1979, hsa-miR-19A, hsa-miR-19B, hsa-miR-20A, hsa-miR-20B, hsa-miR-21, hsa-miR-205, hsa-miR-210, hsa-miR-215, hsa-miR-22, hsa-miR-22*, hsa-miR-221, hsa-miR-222, hsa-miR-223, hsa-miR-223*, hsa-miR-23A, hsa-miR-23B, hsa-miR-24, hsa-miR-25, hsa-miR-26A, hsa-miR-26B, hsa-miR-27A, hsa-miR-27B, hsa-miR-28-5P, hsa-miR-29A, hsa-miR-29B, hsa-miR-29C, hsa-miR-30A, hsa-miR-301A, hsa-miR-30B, hsa-miR-30C, hsa-miR-30D, hsa-miR-30E, hsa-miR-320A, hsa-miR-320B, hsa-miR-324-3P, hsa-miR-326, hsa-miR-328, hsa-miR-338-3P, hsa-miR-342-3P, hsa-miR-339-5P, hsa-miR-33A, hsa-miR-342-3P, hsa-miR-365, hsa-miR-378, hsa-miR-423-3P, hsa-miR-423-5P, hsa-miR-424, hsa-miR-425, hsa-miR-451, hsa-miR-484, hsa-miR-486-5P, hsa-miR-505, hsa-miR-502-3P, hsa-miR-590-5P, hsa-miR-628-3P, hsa-miR-652, hsa-miR-660, hsa-miR-720, hsa-miR-92A, hsa-miR-92B, hsa-miR-93, hsa-miR-93*, hsa-miR-99A, hsa-miR-99B, hsa-miR-103-2*, hsa-miR-106B*, hsa-miR-133A, hsa-miR-133B, hsa-miR-338-3P, hsa-miR-340, hsa-miR-34A, hsa-miR-34B, hsa-miR-376A, hsa-miR-532-3P, hsa-miR-125A-5P, hsa-miR-154, hsa-miR-196B, hsa-miR-1979, hsa-miR-326, hsa-miR-425*, hsa-miR-127-3P, hsa-miR-1537, hsa-miR-183, hsa-miR-29B-2*, hsa-miR-339-3P, hsa-miR-551A, hsa-miR-629, hsa-miR-766, hsa-miR-2110, hsa-miR-361-3P, hsa-miR-501-5P, hsa-miR-940, hsa-miR-1249, hsa-miR-132, hsa-miR-1538, and hsa-miR-149, hsa-miR-125a-5P, hsa-miR-132, hsa-miR-155, hsa-miR-182, hsa-miR-324-5P, hsa-miR-331-3P, hsa-miR-335, hsa-miR-374b, and hsa-miR-532-5P, hsa-miR-7, hsa-miR-320a, hsa-miR-130a, hsa-miR-320c, hsa-miR-628-3p, hsa-miR-637, hsa-miR-320b, hsa-miR-129-5p, hsa-miR-943, hsa-miR-185*, hsa-miR-340*, hsa-miR-744, hsa-miR-638, hsa-miR-585, hsa-miR-26b, hsa-miR-485-3p, hsa-miR-103, hsa-miR-146b-5p, hsa-miR-642, hsa-miR-146a, hsa-let-7a, hsa-let-7f, hsa-miR-200b*, hsa-miR-320d, hsa-let-7d, hsa-miR-1282, hsa-miR-124, hsa-miR-602, hsa-let-7g, hsa-miR-221, hsa-miR-25*, hsa-miR-1184, hsa-miR-663, hsa-miR-93, hsa-miR-30b*, hsa-miR-124*, hsa-miR-22, hsa-miR-1281, hsa-miR-1237, hsa-miR-34b, hsa-miR-1290, hsa-miR-193b*, hsa-miR-526b, hsa-miR-622, hsa-miR-191, hsa-miR-142-3p, hsa-miR-92a, hsa-miR-1280, hsa-miR-1236, hsa-miR-30c, hsa-miR-877*, hsa-miR-548n, hsa-miR-1249, hsa-let-7i, hsa-miR-1224-3p, hsa-miR-17, hsa-miR-300, hsa-miR-193a-5p, hsa-let-7d*, hsa-miR-24, hsa-miR-518c*, hsa-miR-222, hsa-miR-664, hsa-miR-130b, hsa-miR-625*, hsa-miR-593, hsa-miR-885-5p, hsa-miR-505*, hsa-miR-491-3p, hsa-miR-421, hsa-miR-7, hsa-miR-106a, hsa-miR-99b*, hsa-miR-1300, hsa-miR-92b, hsa-miR-30d, hsa-miR-720, hsa-miR-1260, hsa-miR-425, hsa-miR-939, hsa-miR-30a, hsa-miR-30e, hsa-miR-654-5p, hsa-miR-509-5p, hsa-miR-1826 and variants thereof.

As used herein, the term "miRNA" or "miR" or "microRNA" means a non-coding RNA between 17 and 25 nucleobases in length which hybridizes to and regulates the expression of a coding RNA. A 17-25 nucleotide miRNA molecule can be obtained from a miR precursor through natural processing routes (e.g., using intact cells or cell lysates) or by synthetic processing routes (e.g., using isolated processing enzymes, such as isolated Dicer, Argonaut, or RNAase III). It is understood that the 17- 25 nucleotide RNA molecule can also be produced directly by biological or chemical syntheses, without having been processed from a miR precursor.

As used herein the term "miRNA molecule" refers to any nucleic acid molecule representing a miRNA. Included within this definition is natural miRNA molecules, pre-miRNA, pri-miRNA, miRNA molecules identical in nucleic acid sequence to the natural forms as well as nucleic acid sequences, wherein one or more nucleic acids has been replaced or is represented by one or more DNA nucleotide and/or nucleic acid analogue. miRNA molecules is in the present specification occasionally refered to as a nucleic acid molecule encoding a miRNA or simply nucleic acid molecule.

As used herein, the term "miR precursor," "pre-miRNA" or "pre-miR" means a non-coding RNA having a hairpin structure, which contains a miRNA. In certain embodiments, a pre-miRNA is the product of cleavage of a primary mi-RNA transcript, or "pri-miR" by the doublestranded RNA-specific ribonuclease known as Drosha, but a pre-miRNAs can also be produced directly by biological or chemical synthesis without having been processed from a pri-miR.

The term "conditioned cell culture medium" as used herein refers to a medium, such as a growth medium wherein cells have been cultured for a period of time, such as by in vitro cultivation. The period of time for culturing may be 1, 2, 4, 8, 16, 24, 48, 72 hours or as long as the cells are viable and stabile.

Bone marrow exudates may be obtained by bone marrow aspiration, wherein an amount of bone marrow (such as from the hip) is removed through a needle. The needle is placed through the top layer of bone and a liquid sample containing bone marrow cells is obtained by aspirating it into a syringe. The bone marrow exudates may further be centrifuged to obtain a fraction containing blood cells.

The terms "activation", "activated" or "conditioned" as used herein refers to the treatment of a body fluid or element thereof in vitro or in vivo, for a period of time in a container comprising a surface, such as a surface that is able to trigger an immunological response in the leukocytes, such as monocytes or dendritic cells of a blood preparation. In some embodiment whole blood is activated by exposure to an enhancing agent, or by stimulation to express an enhancing agent.

The term "autologous" as used herein refers to a preparation of a composition that are administered to the same individual as they come from. In a preferred embodiment, the body fluid composition is autologous to the mammal in need of said treatment.

### Indications:

Particularly suitable diseases or disorders that may be treated according to the invention may be conditions of the respiratory system, such as respiratory problems, including including breathing, airway or lung problems, such as lower respiratory tract disorders selected from Recurrent Airway Obstruction (RAO) also known as Heaves, Chronic Obstructive Pulmonary Disease (COPD), Inflammatory Airway Disease (IAD), Exercise-Induced Pulmonary Haemorrhage (EIPH), cystic fibrosis, and asthma.

As used herein, the phrase "alleviating the symptoms" means a lessening of severity of at least one indicator of a condition or disease. In certain embodiments, alleviating the symptoms includes a delay or slowing in the progression of one or more indicators of a condition or disease. The severity of indicators may be determined by subjective or objective measures which are known to those skilled in the art.

In the present context, the term "treatment" is meant to include both prevention of an expected condition associated with inflammation, a disease of the immune system, such as undesirable activation of the immune system, cancer or other indications associated with abnormal cell growth or cell division.

Prophylactic administration of the body fluid compositions according to the present invention is thus included in the term "treatment".

The term "subject" as used herein is intended to mean any animal, in particular mammals, such as humans and horses, and may, where appropriate, be used interchangeably with the term "patient". Accordingly, in some embodiments, the subject is a horse. In other embodiments, the subject is a human.

As used herein, the term "cancer" includes, but is not limited to, solid tumors and blood borne tumors, including leukemia and lymphoma. The term cancer refers to diseases of the skin, tissues, organs, bone, cartilage, blood and vessels. The term "cancer" further encompasses primary and metastatic cancers.

### Devices:

Suitable devices to be used according to the present invention include any device suitable for administration of therapeutic compounds to the lungs, such as by inhalation, such as inhalers, including metered-dose inhalers, dry powder inhalers and nebulizers. A suitable nebulizer for delivery of compounds according to the invention to a horse is the Flexineb™ nebulizer, which is a fast, portable equine nebuliser device that is simple to use and noise free (Nortev Nortev Ltd. Co. galway, Ireland).

### Combinations:

Optionally the body fluid composition according to the present invention further comprises a second or a further therapeutic substance, such as another immunostimulatory substance enhancing agent, a diluent, including saline, an agent that facilitate better penetration of active compounds over the mucosal lining of the lungs, such as DMSO, a preparation of cells including stem cells, such as adipose-derived stem cells, or a second or further different body fluid composition.

This further compound may be any asthma medicine, such as Advair, Albuterol, mometasone furoate, fluticasone propionate, Pulmicort, beta agonists, such as long-acting beta agonists, beclamethasone dipropionate, methylxanthines, budesonide, formoterol, Xolair, Xopenex, or any other antibiotic, bronchodilator, corticosteroid, such as Prednisone, or other natural solutions such as: Balsamic Air, Saline, Eucalyptus, Nebulin or EquiSilver.

The body fluid composition according to the present invention may be diluted in any suitable pharmaceutically acceptable diluent including a saline solution.

### Specific embodiments of the invention

The present invention relates to a body fluid composition being a blood serum aerosol for use in the treatment or for alleviating the symptoms of a disease or disorder by the inhalation of said blood serum aerosol to the respiratory system.

The body fluid composition comprises a therapeutically effective amount of one or more miRNA molecule(s) said miRNA being upregulated in said body fluid or element thereof from where said body fluid composition is derived upon activation. In some embodiments the body fluid composition comprises a therapeutically effective amount of one or more miRNA molecule(s) said miRNA being upregulated upon activation. In some embodiments the miRNA is upregulated in a blood preparation upon activation. In some embodiments the miRNA is upregulated in a body fluid or element thereof upon activation in a vessel optionally comprising an inductor. In some embodiments the miRNA is upregulated upon activation on a surface selected from the group consisting of: spheres, gels, glass wool, granulated material and particles or surface structures comprising polystyrene or glass.

The body fluid composition is derived from blood serum.

In some embodiments the body fluid composition according to the present invention further comprises a pharmaceutically acceptable carrier suitable for use in humans.

The body fluid composition according to the present invention is an aerosol. In some embodiments the aerosol contain aerosolized particles having an average aerodynamic diameter of less than about 10 microns, such as from about 1 to about 5 microns.

The disease or disorder being treated or wherein the symptoms are alleviating by the body fluid compositions according to the present invention is selected from respiratory fibrosis, a respiratory hypersensitivity disorder, a cancer, such as lung cancer, non-small cell lung cancer (NSCLC), pulmonary hypertension, chronic obstructive lung disease (COLD), also known as Chronic Obstructive Pulmonary Disease (COPD), Recurrent Airway Obstruction (RAO) also known as Heaves, Inflammatory Airway Disease (IAD), Exercise-Induced Pulmonary Haemorrhage (EIPH), cystic fibrosis, and asthma. In some embodiments the respiratory fibrosis is selected from pulmonary fibrosis, idiopathic pulmonary fibrosis, chronic obstructive pulmonary disease, and chronic asthma. In some embodiments the respiratory hypersensitivity disorder is selected from allergic rhinitis, allergic sinusitis, and allergic asthma.

In some embodiments the body fluid composition is autologous to the subject in need of said treatment.

In some embodiments the body fluid composition is homologous to the subject in need of said treatment.

In some embodiments the body fluid composition is a heterologous to the subject in need of said treatment.

The body fluid composition may further comprise a second or a further therapeutic substance, such as another immunostimulatory substance, an enhancing agent, a diluent, including saline, an agent that facilitate better penetration of active compounds over the mucosal lining of the lungs, such as DMSO, a preparation of cells including stem cells, such as adipose-derived stem cells, or a second or further different body fluid composition.

In some embodiments the subject is a human. In some embodiments the subject is a horse, such as a race horse.

The disease or disorder is selected from respiratory fibrosis, respiratory hypersensitivity disorder, a cancer, such as lung cancer, non-small cell lung cancer (NSCLC), pulmonary hypertension, chronic obstructive lung disease (COLD), also known as Chronic Obstructive Pulmonary Disease (COPD), Recurrent Airway Obstruction (RAO) also known as Heaves, Inflammatory Airway Disease (IAD), Exercise-Induced Pulmonary Haemorrhage (EIPH), cystic fibrosis, and asthma. In some embodiments the respiratory fibrosis is selected from pulmonary fibrosis, idiopathic pulmonary fibrosis, chronic obstructive pulmonary disease, and chronic asthma. In some embodiments the respiratory hypersensitivity disorder is selected from allergic rhinitis, allergic sinusitis, and allergic asthma.

In some embodiments the body fluid composition is administered with a nasal delivery device, with a dry powder inhaler, with a jet, ultrasonic, pressurized or vibrating porous plate nebulizer.

In some embodiments the body fluid composition is autologous to the subject in need of said treatment.

In some embodiments the body fluid composition is homologous to the subject in need of said treatment.

In some embodiments the body fluid composition is a heterologous to the subject in need of said treatment.

The present invention further relates to a kit comprising the body fluid composition according to the present invention and an inhalation device. In some embodiments the inhalation device is selected from a nebulizer, a dose inhaler, or a dry powder delivery mechanism.

The present invention further relates to an inhalation device comprising a body fluid composition according to the present invention.

The inhalation device is selected from a metered-dose inhaler; a dry powder inhaler, a nasal delivery device; or a jet, ultrasonic, pressurized or vibrating porous plate nebulizer.

### EXAMPLES

### Example 1:

4 racehorses, suffering from chronic pharyngitis (more than 2 months without successful treatments) characterized by numerous inflamed follicles, were treated once a day for 8 days inhaling 10ml of an atomized mixture of 50% conditioned serum and 50% sterile water. The horses were endoscoped before and after the treatment period. A full recovery to normal was seen in 3 of the 4 horses and an estimated 70% improvement in the 4 horse.

One of the horses had started several times in the past 6 months with very poor performance but won a horse race over 1600 meters 8 days after the treatment.

### Example 2:

6 trotting horses suffering from suboptimal performance due to chronic inflammation in the lungs diagnosed by endoscopy. The horses had an increased number of heartbeats and an increased amount of lactic acid in the blood following a standard training program. Following months (2 - 8 months) of unsuccessful treatments the horses were treated once a day for 8 days inhaling 10ml of an atomized mixture of 50% conditioned serum and 50% sterile water.

After the treatment period all the horses recovered to their normal levels of lactic acid in the blood and had normal heartbeats 15 minutes following the standard training program. The number of heartbeats per minute were on average 6 beats lower (range 4-8). The horses were able to cover 2000 meters of trotting 1-2 seconds faster per km compared to pretreatment. The results of endoscopy of the airways after 8 treatments were normal.

## Claims

1. A body fluid composition being a blood serum aerosol for use in the treatment or for alleviating the symptoms of a disease or disorder by the inhalation of said blood serum aerosol to the respiratory system; wherein said blood serum aerosol comprises a therapeutically effective amount of one or more miRNA molecule(s), said miRNA being upregulated in peripheral blood upon activation of said peripheral blood by a method comprising the steps of incubating said blood collected from a mammal in contact with an increased surface area; and collecting said activated serum produced, wherein said disease or disorder is selected from respiratory fibrosis, a respiratory hypersensitivity disorder, a cancer, such as lung cancer, non-small cell lung cancer (NSCLC), pulmonary hypertension, chronic obstructive lung disease (COLD), also known as Chronic Obstructive Pulmonary Disease (COPD), Recurrent Airway Obstruction (RAO) also known as Heaves, Inflammatory Airway Disease (IAD), Exercise-Induced Pulmonary Haemorrhage (EIPH), cystic fibrosis, and asthma.

2. The body fluid composition for use according to claim 1, wherein said miRNA is upregulated in peripheral blood upon activation in a vessel optionally comprising an inductor.

3. The body fluid composition for use according to any one of claims 1-2, wherein said miRNA is upregulated upon activation on a surface selected from the group consisting of: spheres, gels, glass wool, granulated material and particles or surface structures comprising polystyrene or glass.

4. The body fluid composition for use according to any one of claims 1-3, wherein the aerosol contain aerosolized particles having an average aerodynamic diameter of less than about 10 microns, such as from about 1 to about 5 microns.

5. The body fluid composition for use according to claim 1, wherein said respiratory fibrosis is selected from pulmonary fibrosis, idiopathic pulmonary fibrosis, chronic obstructive pulmonary disease, and chronic asthma.

6. The body fluid composition for use according to claim 1, wherein said respiratory hypersensitivity disorder is selected from allergic rhinitis, allergic sinusitis, and allergic asthma.

7. The body fluid composition for use according to any one of claims 1-6, wherein said body fluid composition is autologous to the subject in need of said treatment.

8. The body fluid composition for use according to any one of claims 1-6, wherein said body fluid composition is homologous to the subject in need of said treatment.

9. The body fluid composition for use according to any one of claims 1-6, wherein said body fluid composition is a heterologous to the subject in need of said treatment.

10. The body fluid composition for use according to any one of claims 1-9, wherein the formulation is a part of a kit comprising said composition and an inhalation device.

11. The body fluid composition for use according to any one of claims 1-9, wherein the formulation is comprised in an inhalation device.

## Patentansprüche

1. Körperflüssigkeitszusammensetzung, die ein Blutserumaerosol darstellt, zur Verwendung bei der Behandlung oder zur Linderung der Symptome einer Krankheit oder Störung durch Inhalation des Blutserumaerosols in die Atemwege; wobei das Blutserumaerosol eine therapeutisch wirksame Menge eines oder mehrerer miRNA-Moleküle umfasst, wobei die miRNA im peripheren Blut hochreguliert wird bei Aktivierung des peripheren Blutes durch ein Verfahren, das die Schritte umfasst des Inkubierens des von einem Säugetier gesammeltem Bluts in Kontakt mit einer vergrößerten Oberfläche; und des Sammelns des produzierten aktivierten Serums, wobei die Krankheit oder Störung ausgewählt ist aus einer Fibrose der Atemwege, einer Atemwegsüberempfindlichkeitsstörung, einem Krebs, wie etwa Lungenkrebs, nicht-kleinzelligem Lungenkrebs (NSCLC), pulmonaler Hypertonie, chronisch obstruktiver Lungenerkrankung (COLD), auch bekannt als chronisch obstruktive Pulmonarerkrankung (COPD), rezidivierende Atemwegsobstruktion (RAO), auch bekannt als "Heaves", entzündlicher Atemwegserkrankung (IAD), belastungsinduzierter Lungenblutung (EIPH), Mukoviszidose und Asthma.

2. Körperflüssigkeitszusammensetzung zur Verwendung nach Anspruch 1, wobei die miRNA bei Aktivierung in einem Blutgefäß, das wahlweise einen Induktor umfasst, im peripheren Blut hochreguliert wird.

3. Körperflüssigkeitszusammensetzung zur Verwendung nach einem der Ansprüche 1-2, wobei die miRNA bei Aktivierung auf einer Oberfläche hochreguliert wird, die ausgewählt ist aus der Gruppe, bestehend aus: Kugeln, Gelen, Glaswolle, granuliertem Material und Partikeln oder Oberflächenstrukturen, die Polystyrol oder Glas umfassen.

4. Körperflüssigkeitszusammensetzung zur Verwendung nach einem der Ansprüche 1-3, wobei das Aerosol aerosolisierte Partikel mit einem durchschnittlichen aerodynamischen Durchmesser von weniger als etwa 10 Mikrometer enthält, wie etwa 1 bis etwa 5 Mikrometer.

5. Körperflüssigkeitszusammensetzung zur Verwendung nach Anspruch 1, wobei die die Fibrose der Atemwege ausgewählt wird aus Lungenfibrose, idiopathischer Lungenfibrose, chronisch obstruktiver Lungenerkrankung und chronischem Asthma.

6. Körperflüssigkeitszusammensetzung zur Verwendung nach Anspruch 1, wobei die Atemwegsüberempfindlichkeitsstörung ausgewählt ist aus allergischer Rhinitis, allergischer Sinusitis und allergischem Asthma.

7. Körperflüssigkeitszusammensetzung zur Verwendung nach einem der Ansprüche 1-6, wobei die Körperflüssigkeitszusammensetzung zu dem Subjekt, das die Behandlung benötigt, autolog ist.

8. Körperflüssigkeitszusammensetzung zur Verwendung nach einem der Ansprüche 1-6, wobei die Körperflüssigkeitszusammensetzung zu dem Subjekt, das die Behandlung benötigt, homolog ist.

9. Körperflüssigkeitszusammensetzung zur Verwendung nach einem der Ansprüche 1-6, wobei die Körperflüssigkeitszusammensetzung zu dem Subjekt, das die Behandlung benötigt, heterolog ist.

10. Körperflüssigkeitszusammensetzung zur Verwendung nach einem der Ansprüche 1-9, wobei die Formulierung ein Teil eines Kits ist, der die Zusammensetzung und eine Inhalationsvorrichtung umfasst.

11. Körperflüssigkeitszusammensetzung zur Verwendung nach einem der Ansprüche 1-9, wobei die Formulierung in einer Inhalationsvorrichtung enthalten ist.

## Revendications

1. Composition de fluide corporel étant un aérosol de sérum sanguin pour son utilisation dans le traitement ou le soulagement des symptômes d'une maladie ou d'un trouble par l'inhalation dudit aérosol de sérum sanguin dans le système respiratoire ; dans laquelle ledit aérosol de sérum sanguin comprend une quantité thérapeutiquement efficace d'une ou plusieurs molécules de miARN, ledit miARN étant régulé à la hausse dans le sang périphérique lors de l'activation dudit sang périphérique par un procédé comprenant les étapes d'incubation dudit sang prélevé d'un mammifère en contact avec une surface accrue ; et de prélèvement dudit sérum activé produit, dans laquelle ladite maladie ou ledit trouble est sélectionné parmi la fibrose respiratoire, un trouble d'hypersensibilité respiratoire, un cancer, par exemple le cancer du poumon, le cancer du poumon non à petites cellules (NSCLC), l'hypertension pulmonaire, une maladie pulmonaire chronique obstructive (COLD), également connue comme la bronchopneumopathie chronique obstructive (COPD), une obstruction récurrente des voies respiratoires (RAO) également connue comme l'emphysème pulmonaire alvéolaire, une maladie inflammatoire des voies respiratoires (IAD), une hémorragie pulmonaire induite par l'exercice (EIPH), la mucoviscidose et l'asthme.

2. Composition de fluide corporel pour son utilisation selon la revendication 1, dans laquelle ledit miARN est régulé à la hausse dans le sang périphérique lors de l'activation dans un vaisseau comprenant facultativement un inducteur.

3. Composition de fluide corporel pour son utilisation selon l'une quelconque des revendications 1 et 2, dans laquelle ledit miARN est régulé à la hausse lors de l'activation sur une surface sélectionnée dans le groupe consistant en : des sphères, des gels, de la laine de verre, un matériau granulé et des particules ou des structures de surface comprenant du polystyrène ou du verre.

4. Composition de fluide corporel pour son utilisation selon l'une quelconque des revendications 1 à 3, dans laquelle l'aérosol contient des particules aérosolisées ayant un diamètre aérodynamique moyen inférieur à environ 10 microns, par exemple d'environ 1 à environ 5 microns.

5. Composition de fluide corporel pour son utilisation selon la revendication 1, dans laquelle ladite fibrose respiratoire est sélectionnée parmi la fibrose pulmonaire, la fibrose pulmonaire idiopathique, la bronchopneumopathie chronique obstructive, et l'asthme chronique.

6. Composition de fluide corporel pour son utilisation selon la revendication 1, dans laquelle ledit trouble d'hypersensibilité respiratoire est sélectionné parmi la rhinite allergique, la sinusite allergique et l'asthme allergique.

7. Composition de fluide corporel pour son utilisation selon l'une quelconque des revendications 1 à 6, dans laquelle ladite composition de fluide corporel est autologue au sujet nécessitant ledit traitement.

8. Composition de fluide corporel pour son utilisation selon l'une quelconque des revendications 1 à 6, dans laquelle ladite composition de fluide corporel est homologue au sujet nécessitant ledit traitement.

9. Composition de fluide corporel pour son utilisation selon l'une quelconque des revendications 1 à 6, dans laquelle ladite composition de fluide corporel est hétérologue au sujet nécessitant ledit traitement.

10. Composition de fluide corporel pour son utilisation selon l'une quelconque des revendications 1 à 9, dans laquelle la formulation fait partie ou un kit comprenant ladite composition et un dispositif d'inhalation.

11. Composition de fluide corporel pour son utilisation selon l'une quelconque des revendications 1 à 9, dans laquelle la formulation est comprise dans un dispositif d'inhalation.
